Europäisches Patentamt

European Patent Office

Office européen des brevets

(19)

(11) Publication number: 0 165 757

A2

(12) **EUROPEAN PATENT APPLICATION**

(21) Application number: 85304128.3

(22) Date of filing: 11.06.85

(51) Int. Cl.⁴: **C 12 P 13/22**
//C12N9/88

(30) Priority: 11.06.84 US 619117

(43) Date of publication of application:
27.12.85 Bulletin 85/52

(84) Designated Contracting States:
AT BE CH DE FR GB IT LI LU NL SE

(71) Applicant: GENEX CORPORATION
6110 Executive Boulevard
Rockville Maryland 20852(US)

(72) Inventor: Swann, Wayne Elliott
5392 Storm Drift
Columbia, Maryland 20877(US)

(74) Representative: Hildyard, Edward Martin et al,
Frank B. Dehn & Co. European Patent Attorneys Imperial
House 15-19 Kingsway
London WC2B 6UZ(GB)

(54) Production of L-phenylalanine.

(57) A method for producing L-phenylalanine by reacting trans-cinnamic acid with ammonium ions in the presence of phenylalanine ammonia-lyase and recovering the L-phenylalanine, wherein said reaction is performed at a temperature below about 30°C.

EP 0 165 757 A2

## "PRODUCTION OF L-PHENYLALANINE"

### Background of the Invention

Enzymatic methods using L-phenylalanine ammonia-lyase for the conversion of trans-cinnamic acid to L-phenylalanine generally comprise the steps of (a) aerobically propagating a phenylalanine ammonia-lyase (hereinafter PAL)-producing microorganism in an aqueous nutrient medium until substantial amounts of PAL are produced, (b) contacting the cells of the PAL-producing microorganism from step (a), either as the whole culture broth or separated cells therefrom, or the isolated enzyme, with ammonium ions and trans-cinnamate ions and allowing the reaction to proceed under controlled temperature and pH conditions until the conversion to L-phenylalanine approaches equilibrium and (c) separating and recovering the L-phenylalanine from the reaction mixture.

The foregoing method is described, for example, in British Patent No. 1,489,468 (October 19, 1977). A drawback to the use of this process for commercial production has been the relative instability of PAL, and its inhibition by the substrate, t-cinnamic acid. To drive the reaction toward the production of L-phenylalanine and to counteract the effects of substrate inhibition, the above-mentioned British patent describes a process which employs large masses of PAL-containing cells and excess concentrations of ammonium ions.

Yamada, S. et al. (Appl. and Environ. Microbiol., 42, 7873-778 (1981)) have described the production of L-phenylalanine from t-cinnamic acid using PAL-containing Rhodotorula glutinis cells. They speculated that the lack of previous practical application of this process was attributable to the low activity and instability of microbial PAL. Yamada, et al. found that L-isoleucine had a stabilizing effect on PAL, and extended the useful period of activity of the enzyme. These authors further observed the inhibitive effect of the substrate, noting that at practical concentrations of t-cinnamic acid (150 mM), the rate of conversion of L-phenylalanine was reduced to one-half the maximum rate. Maximum yields obtained were in the range of about 70%, and the authors note that higher yields were not observed even when a large excess of cells were employed.

## Summary of the Invention

In accordance with the present invention, an improved process for producing L-phenylalanine involves reacting trans-cinnamic acid with ammonium ions in the presence of phenalalanine ammonia lyase at a temperature below about 30°C and recovering the L-phenylalanine.

It has been found that the yield of L-phenylalanine can be substantially increased by lowering the reaction temperature during at least the final stages of the reaction process.

## Description of the Invention

In the practice of this invention, the process of making L-phenylalanine by reacting trans-cinnamic acid and ammonium ions  in the presence of L-phenylalanine ammonia-lyase has been improved, such that higher yields of L-phenylalanine are obtained.

This improvement involves conducting the enzymatic conversion reaction at temperatures lower than those used previously. It has been discovered that, when the reaction is run at temperatures below about 30°C, the yield of L-phenylalanine is enhanced. At lower temperatures, equilibrium favors the product L-phenylalanine, but the rate of reaction, dependent on kinetics, is slower. A higher yield is thereby obtained over a longer period of time. At temperatures below 30°C, yields of 90% or greater can be obtained, whereas yields are considerably lower under similar conditions when the temperature is increased. Low temperature operation provides the added advantage of increased enzyme stability and the ability to reuse enzyme-containing cells a greater number of times.

If desired, the conversion reaction can be conducted in two stages, wherein the first stage is carried out at a relatively high temperature which is favorable to a high rate of reaction. The second stage, conducted at a lower temperature, is optimized for a high yield of L-phenylalanine. A majority of the conversion occurs rapidly in the first stage, where temperature-dependent reaction kinetics predominate. As equilibrium is approached at about a 70% conversion, the temperature is advantageously reduced to the lower value of the second reaction stage, where equilibrium can be reached at an L-phenylalanine conversion of 90% or greater. It should be recognized, however, that the use of higher initial reaction temperatures may reduce the useful lifetime of the enzyme.

Generally, the conversion utilizes PAL-producing microorganisms which have previously been cultivated under aerobic, growth-promoting conditions. Conventional procedures are employed for growing the cells. Cells are inoculated into a nutritional medium containing assimilable sources of carbon and nitrogen and essential vitamins, minerals and other growth factors. Suitable carbon sources can include various refined or crude carbohydrates such as glucose, sucrose, molasses, starches, grains and the like. A preferred carbon source is glucose. Nitrogen sources include inorganic ammonium salts, such as ammonium phosphate, ammonium sulfate, ammonium acetate, ammonium citrate, ammonium nitrate and the like and organic nitro-geneous substances such as soybean meal, meat infusions, amino acids, corn steep liquor, protein hydrolyzates, peptone, yeast extracts, and the like. A preferred nitrogen source for the process of this invention is yeast extract, and this nutrient may advantageously be combined with diammonium phosphate which supplies both nitrogen and phosphorous.

Vitamins, minerals and other growth factors may be supplied by the carbon and nitrogen sources (e.g., via the yeast extract) or may be supplied separately. These components can vary with the particular microorganism employed. Typically, trace minerals such as zinc, manganese, iron, cobalt, and calcium can be supplied in growth-promoting amounts as inorganic salts. These minerals may, for example, be supplied with process water, e.g., tap water, sea water, etc. Nutrient media of the type described are well known, and can vary in composition widely.

After growing the cells to the desired cell density under aerobic conditions, they are induced to make PAL under aerobic, PAL-producing conditions. PAL induction is generally achieved by adding small amounts of a compound that acts as a substrate for the PAL. L-Phenylalanine is a good PAL inducer, and a number of analogs of L-phenylalanine also induce the synthesis of this enzyme. For example, D,L-phenylalanine, L-tyrosine, and D,L-tyrosine can be employed for this purpose. In addition, it has been discovered that various crude nitrogen sources can be used for PAL induction. Such crude nitrogen sources include hydrolyzed proteins which contain substantial amounts of L-phenylalanine or L-tyrosine. Casein and blood hydrolyzates can advantageously be used as crude nitrogen sources for the induction of PAL synthesis.

The PAL inducer is added to the cells in a PAL-inducing amount, which generally ranges from about 0.1 to 10 g/l of the fermentation medium. Preferably, the PAL inducer is employed at a concentration from about 4 to about 8 g/l of the fermentation medium. During this step, PAL-inducing conditions of temperature and pH, aeration and agitation are maintained. The temperature and pH are generally maintained within physiologically compatible limits during PAL induction. Somewhat reduced temperatures, e.g. from about 15°C to about 25°C are preferred, because at these lower temperatures, enzyme stability is improved and the rate of consumption of the PAL inducer is decreased. A preferred pH for the PAL induction ranges from about 5.5 to about 7.5, where relatively higher PAL levels are achieved.

. If the cells employed are sensitive to catabolic repression of PAL synthesis, then, prior to induction, means should be employed to reduce or eliminate catabolites and their precursors from the medium. This may be accomplished by separating cells from the medium, washing them and suspending them in a catabolite-free medium. Alternatively, the cells can be allowed to grow until the nutrients are substantially exhausted before the PAL induction procedure is initiated.

The cells are advantageously cultivated under PAL-inducing conditions until the PAL activity reaches at least about 0.5 units per ml, preferably at least about 2.0 units per ml. A unit of PAL activity is defined as the amount of enzyme which catalyzes the formation of 0.83 μmole of t-cinnamic acid per minute at 22°C or 1 μmole per minute at 30°C. It has been observed that under these conditions, the PAL activity increases to a certain point and then begins to diminish. PAL produced by these procedures may be employed to produce L-phenylalanine from t-cinnamic acid and ammonia. These reactants can be added directly to the PAL-containing cells in an aqueous medium, or the cells or enzyme isolated therefrom can be immobilized by known procedures on a solid support that can be reused for so long as the enzyme activity is maintained.

L-Phenylalanine is produced by this method under L-phenylalanine-producing conditions. These conditions will vary, depending upon the particular microbial strains employed, whether whole cells or cell-free enzyme preparations are used and whether immobilized systems are employed. The PAL-catalyzed reaction of t-cinnamic acid and ammonia to produce L-phenylalanine is reversible, and in fact, the equilibrium favors the breakdown of L-phenylalanine. Therefore, another factor besides temperature which affects reaction rate is the concentration of reactants. To establish a favorable reaction rate, the t-cinnamic acid concentration in the reaction mixture is advantageously maintained at a relatively high level. On the other hand, excessive concentrations of t-cinnamic acid inhibit the activity of PAL. Accordingly, t-cinnamic acid can periodically or continuously be fed into the reaction mixture during all but the latter stage of the reaction to maintain the concentration of this reactant within the ranges referred to above. The inhibitory effect of ammonium ions on PAL is somewhat less than t-cinnamic acid and therefore the concentration of ammonium ions in many prior art conversion processes is substantially in excess of stoichiometric in order to increase L-phenylalanine yield. At the lower reaction temperatures of the present invention, however, a large excess of ammonium ions is unnecessary. In general, t-cinnamic acid and aqueous ammonia (or soluble ammonium salts) are supplied in amounts such that the ammonia is in excess of the t-cinnamic acid on a molar basis. The t-cinnamic acid is provided to the reaction medium in concentrations of from about 0.01 M to about 1.0 M, preferably from about 0.1 M to about 0.5 M. At these t-cinnamic acid concentrations, the ammonia concentration generally ranges from about 0.1 to about 10.0 molar, preferably from about 1.0 to about 8.0 molar.

Our copending U.S. Patent Application, Serial No. 619,183, filed June 11, 1984 describes an alternative method of feeding t-cinnamic acid to the reaction in controlled concentrations. A two-phase bioreaction mixture is prepared containing an ester of cinnamic acid, e.g., methyl cinnamate in the nonaqueous phase and ammonium ions in the aqueous phase. The bioreaction mixture is maintained under temperature and solvent conditions in which a small amount of the cinnamic acid ester is dissolved in the aqueous phase at a desired concentration. The aqueous phase is contacted with an enzyme which de-esterifies the dissolved ester and with phenylalanine ammonia-lyase, so that the ammonium ions react with t-cinnamic acid in the aqueous phase to produce L-phenylalanine. As the cinnamic acid ester is de-esterified and converted to L-phenylalanine in the aqueous phase it is replenished by cinnamic acid ester which passes into solution from the nonaqueous phase.

Preferred ammonium salts are those which contain no halogen ions. The presence of halogens in the substrate solution has been found to inhibit the catalytic activity of PAL. Therefore, preferred ammonium salts include ammonium carbonate, ammonium sulfate, ammonium nitrate, ammonium citrate, ammonium acetate, and ammonium phosphate. An especially preferred ammonium salt is ammonium carbonate. A convenient procedure for preparing a substrate solution is to dissolve the t-cinnamic acid in an aqueous ammonia solution, and then adjust the pH of the solution as desired by sparging with carbon dioxide or adding a mineral acid, such as sulfuric acid.

The enzymatic conversion of t-cinnamic acid and ammonia to L-phenylalanine is preferably conducted in a bioreactor vessel. This procedure advantageously involves separating the cells from their fermentation medium by filtration or centrifugation and suspending them in the substrate solution of t-cinnamic acid and ammonium ions. Alternatively, the cells or the PAL can be immobilized on a solid support and the conversion reaction run continuously.

The bioreaction mixture is maintained at a temperature of from about 0°C to about 30°C at least through the last portion of the conversion process. Preferably the temperature is from about 5°C to about 25°C. If desired, the reaction can be run at a somewhat higher temperature, e.g., above about 25°C during the initial stage of the reaction to increase the reaction rate. The temperature is then reduced, preferably to about 5°C to 25°C, during the final stages of reaction to increase the yield. Typically, the reaction is run at the higher temperature until conversion approaches about 70%.

The bioreaction is continued until substantial amounts of L-phenylalanine have accumulated in the reaction mixture. L-Phenylalanine can be recovered from the reaction mixture by any suitable means. For example, solids can be removed by filtration or centrifugation to produce a clarified solution, and L-phenylalanine can be precipitated from that solution by adjusting the pH to the isoelectric point of L-phenylalanine, i.e., about 5.5

The following examples further illustrate the present invention, and demonstrate the beneficial effects of reduced temperature during the conversion reaction. The invention, however, is not to be construed as being limited by the examples. In the examples, the abbreviation "C.A." is used for t-cinnamic acid.

## Example 1

A continuous, single-pass, L-phenylalanine conversion reaction was conducted using a plug-flow column bioreactor containing whole cells of R. rubra immobilized on vermiculite. The following reaction conditions were employed:

## Reaction Conditions

| | |
|---|---|
| pH | 10.3 |
| Temperature | 20°C |
| [1] Flow rate ($SV^{h-1}$) | 0.04 |
| PAL activity (U/gm dry weight of cells) | 90 |

## Feed Stream

| | |
|---|---|
| Total ammonia (as ammonium carbonate and ammonium hydroxide) | 7.5 M |
| C.A. (41.1 gm/l) | 0.278 M |

## Effluent Stream

| | |
|---|---|
| [2]C.A. (U.V. absorbance) | 0.0336 M |
| [2]L-PHE (L-AAO assay) | 0.2667 M |

---

[1] $SV^{h-1}$ = reactor volumes per hour
[2] Average values taken on 4 consecutive days

The effluent measurements of 266.7 mM L-phenylalanine and 33.6 mM cinnamic gave a conversion rate of 88.8%. Conversion was based on effluent concentration of cinnamic acid, rather than on the concentration in the feed stream, because the support material in the column has a slight affinity for cinnamic acid in the early stages of the operation. Consequently, feed stream concentration is not a true measure of substrate available to the reaction. The apparent conversion rate would have been higher (95.9%) if it had been based on cinnamic acid concentration in the feed stream. The Keq was calculated to be 1.19 under these reaction conditions.

## Example 2

A continuous, single-pass L-phenylalanine conversion reaction was conducted, in a manner similar to that described in Example 1, using somewhat higher concentrations of cinnamic acid and ammonia in the feed stream. Reaction conditions were as follows:

### Reaction Conditions

| | |
|---|---|
| pH | 10.3 |
| Temperature | 20°C |
| [1] Flow rate ($SV^{h-1}$) | 0.015 |
| PAL activity (U/gm dry weight of cells) | 90 |

### Feed Stream

| | | |
|---|---|---|
| Ammonia added as ammonium carbonate | 2.25 | M |
| Ammonia added as ammonium hydroxide | 5.60 | M |
| Total ammonia | 7.85 | M |
| C.A. (53.98 gm/l) | 0.365 | M |

### Effluent Stream

| | | |
|---|---|---|
| [2]C.A. (U.V. absorbance) | 0.043 | M |
| [2]L-PHE (L-AAO assay) | 0.356 | M |

---

[1]  $SV^{h-1}$ = reactor volumes per hour
[2]  Average values taken on 4 consecutive days

The effluent measurements of 356 mM L-phenylalanine and 43 mM cinnamic acid gave a conversion rate of 89.2% and a Keq of 1.13 under these reaction conditions.

Example 3

To illustrate the effect of reaction temperature on conversion rate, a series of batch reactions was carried out at varying temperatures. In each instance, the reaction was run for 2 days using 6 M ammonia, pH 10.2, 42 mM t-cinnamic acid and 1 gram (dry weight) of R. rubra cells per 50 c.c. of substrate. Samples were taken during the 2-day run and assayed for both L-phenylalanine (L-AAO assay) and t-cinnamic acid (U.V. absorbance). Equilibrium was observed in all reactors by the end of 24 hours. Percentage conversion and Keq were calculated for each reaction temperature. The table below gives equilibrium concentrations of L-phenylalanine and t-cinnamic acid, as well as percentage conversion and Keq at each temperature. The data clearly shows that conversion percentage increases as temperature is reduced.

| Temperature °C | mM L-PHE | mM CA | % Conversion | Keq |
|---|---|---|---|---|
| 12.3 | 37.77 | 4.32 | 89.93 | 1.47 |
| 17.9 | 37.39 | 5.19 | 89.02 | 1.21 |
| 21.1 | 37.03 | 5.80 | 88.17 | 1.07 |
| 26.3 | 35.27 | 6.36 | 83.98 | 0.93 |
| 30.3 | 34.36 | 6.85 | 81.81 | 0.84 |
| 32.9 | 33.82 | 8.21 | 80.52 | 0.69 |

12

## Example 4

Eight batch reactors were run, 4 each at 15°C and 30°C, at pH's of 9.75, 10.00, 10.25 and 10.50. Reactions were run to equilibrium using 40mM CA and 7.5M ammonia with 1 gram (dry weight) of R. rubra cells (90 U/gm) per 50 cc of substrate. Equilibrium concentrations of L-phenylalanine and t-cinnamic acid were determined by L-AAO assay and U.V. absorbance, respectively, and used to calculate Keq. Results are tabulated in Table I. It can be seen that conversion (Keq) to L-phenylalanine is temperature dependent and essentially independent of pH.

| pH | Observed Keq | |
|---|---|---|
| | 15°C | 30°C |
| 9.75 | 1.14 | 0.63 |
| 10.00 | 1.14 | 0.55 |
| 10.25 | 1.14 | 0.55 |
| 10.50 | 1.17 | 0.57 |

CLAIMS:

1. A method for producing L-phenylalanine by reacting trans-cinnamic acid with ammonium ions in the presence of phenylalanine ammonia-lyase and recovering the L-phenylalanine, characterised in

that said reaction is performed at a temperature below about 30°C.

2. A method as claimed in claim 1, wherein the temperature is from about 5°C to about 25°C.

3. A method of producing L-phenylalanine by reacting trans-cinnamic acid with ammonium ions in the presence of phenylalanine ammonia-lyase and recovering the L-phenylalanine characterised in

that a first stage of said reaction is carried out at a relatively high temperature to enhance the reaction rate and the second stage of said reaction is carried out at a lower temperature, which is below 30°C, to increase the yield.

4. A method as claimed in claim 3, wherein the first stage of the reaction is carried out at a temperature of above about 25°C and the second stage is carried out at a temperature of from about 5°C to about 25°C.

5. A method as claimed in claim 3 or 4 wherein said first stage is continued until the point of equilibrium is approached at said relatively high temperature, whereafter the temperature is lowered for said second stage.

6. A method as claimed in claim 5 wherein said point of equilibrium is about 70% conversion to L-phenylalanine.

7. A method as claimed in claim 4, 5 or 6 wherein said second stage is continued until the point of equilibrium is approached at said lower temperature.

8. A method as claimed in claim 7 wherein said point of equilibrium is about 90% conversion to L-phenylalanine.